# EUROPEAN PATENT APPLICATION

(11) **EP 1 250 923 A2**
(43) Date of publication of application: **23.10.2002**
(21) Application number: 02252729.5
(22) Date of filing: 17.04.2002
(51) Int. Cl.: A61K 31/00, A61K 31/472, A61P 25/22, A61P 25/18, A61P 25/14, A61P 25/16, A61P 25/30, A61P 25/32, A61P 25/36, A61P 25/28, A61P 25/24

(54) **Use of selective PDE10 inhibitors for the treatment of central nervous system disorders**

(30) Priority: 20.04.2001 US 285148 P
(71) Applicant: Pfizer Products Inc., Groton, Connecticut 06340 (US)
(72) Inventor: Lebel, Lorraine Ann, Pfizer Global Res. & Dev., Groton, Connecticut 06340 (US); Menniti, Frank Samuel, Pfizer Global Res. & Dev., Groton, Connecticut 06340 (US); Schmidt, Christopher,J. Pfizer Global Res. & Dev., Groton, Connecticut 06340 (US)
(74) Representative: McMunn, Watson Palmer

(57) **Abstract**

The invention provides a method for treating certain neurologic and psychiatric disorders in mammals, including humans, comprising administration of a selective PDE10 inhbitor. In particular, the invention relates to treatment of mood, movement, and anxiety disorders; psychosis; drug, for example alcohol, addiction; and disorders having as a symptom deficient cognition. The invention furthermore provides the use of papaverine as a selective inhibitor of PDE10.

## Description

### Background of the Invention

The subject invention relates to the treatment of disorders of the central nervous system. More particularly, the invention relates to treatment of neurologic and psychiatric disorders, for example psychosis and disorders comprising deficient cognition as a symptom. This invention also relates to PDE10 inhibition.

The cyclic nucleotides, cyclic-adenosine monophosphate (cAMP) and cyclic-guanosine monophosphate (cGMP), function as intracellular second messengers regulating a vast array of intracellular processes including in neurons in the central nervous system. cAMP is synthesized by a family of membrane bound enzymes, the adenylyl cyclases. A broad range of serpin family receptors regulates these enzymes through a coupling mechanism mediated by heterotrimeric G-proteins. Increases in intracellular cAMP leads to activation of cAMP-dependent protein kinases, which regulate the activity of other signaling kinases, transcription factors, and enzymes via their phosphorylation. Cyclic-AMP may also directly affect the activity of cyclic nucleotide regulated ion channels, phosphodiesterases, or guanine nucleotide exchange factors. Recent studies also suggest that intracellular cAMP may function as a precursor for the neuromodulator, adenosine, following its transport out of the cell.

Guanylyl cyclase, which synthesizes cGMP, exists in membrane bound and cytoplasmic forms. The membrane bound form is coupled to G-protein linked receptors such as that for ANP (atrial naturetic peptide) whereas soluble guanylyl cyclase is activated by nitric oxide (Wang, X. and Robinson, P. J. Journal of Neurochemistry 68(2):443-456, 1997). Similar to cAMP, downstream mediators of cGMP signaling in the central nervous system include cGMP-gated ion channels, cGMP-regulated phosphodiesterases and cGMP-dependent protein kinases. Given the important role of cyclic nucleotides in signal transduction within the central nervous system, therapeutic benefits may be derived from the use of compounds that affect the regulation of cyclic nucleotide signaling.

A principal mechanism for regulating cyclic nucleotide signaling is by phosphodiesterase-catalyzed cyclic nucleotide catabolism. There are eleven known families of phosphodiesterases (PDEs) encoded by 21 different genes. Each gene typically yields multiple splice variants that further contribute to the isozyme diversity. The PDE families are distinguished functionally based on cyclic nucleotide substrate specificity, mechanism(s) of regulation, and sensitivity to inhibitors. Furthermore, PDEs are differentially expressed throughout the organism, including in the central nervous system. As a result of these distinct enzymatic activities and localization, different PDEs isozymes can serve distinct physiological functions. Furthermore, compounds that can selectively inhibit distinct PDE families or isozymes may offer particular therapeutic effects, fewer side effects, or both.

PDE10 is identified as a unique family based on primary amino acid sequence and distinct enzymatic activity. Homology screening of EST databases revealed mouse PDE10A as the first member of the PDE10 family of phosphodiesterases (Fujishige et al., J. Biol. Chem. 274:18438-18445, 1999; Loughney, K. et al., Gene 234:109-117, 1999). The murine homologue has also been cloned (Soderling, S. et al., Proc. Natl. Acad. Sci. USA 96:7071-7076, 1999) and N-terminal splice variants of both the rat and human genes have been identified (Kotera, J. et al., Biochem. Biophys. Res. Comm. 261:551-557, 1999; Fujishige, K. et al., Eur. J. Biochem. 266:1118-1127, 1999). There is a high degree of homology across species. The mouse PDE10A1 is a 779 amino acid protein that hydrolyzes both cAMP and cGMP to AMP and GMP, respectively. The affinity of PDE10 for cAMP (Kₘ = 0.05 µM) is higher than for cGMP (Kₘ = 3 µM). However, the approximately 5-fold greater Vₘₐₓ for cGMP over cAMP has lead to the suggestion that PDE10 is a unique cAMP-inhibited cGMPase (Fujishige et al., J. Biol. Chem. 274:18438-18445, 1999).

PDE10 also is uniquely localized in mammals relative to other PDE families. mRNA for PDE10 is highly expressed only in testis and brain (Fujishige, K. et al., Eur J Biochem. 266:1118-1127, 1999; Soderling, S. et al., Proc. Natl. Acad. Sci. 96:7071-7076, 1999; Loughney, K. et al., Gene 234:109-117, 1999). These initial studies indicated that within the brain PDE10 expression is highest in the striatum (caudate and putamen), n. accumbens, and olfactory tubercle. More recently, a detailed analysis has been made of the expression pattern of PDE10 mRNA in rodent brain (Seeger, T.F. et al., Abst. Soc. Neurosci. 26:345.10, 2000).

### Summary of the Invention

The present invention provides a method of treating an anxiety or psychotic disorder in a mammal, including a human, which comprises administering to said mammal an amount of a selective PDE10 inhibitor effective in treating said anxiety or psychotic disorder.

The invention also provides a method of treating an anxiety or psychotic disorder in a mammal, including a human, which comprises administering to said mammal an amount of a selective PDE10 inhibitor effective in inhibiting PDE10.

Examples of psychotic disorders that can be treated according to the present invention include, but are not limited to, schizophrenia, for example of the paranoid, disorganized, catatonic, undifferentiated, or residual type; schizophreniform disorder, schizoaffective disorder, for example of the delusional type or the depressive type; delusional disorder; substance-induced psychotic disorder, for example psychosis induced by alcohol, amphetamine, cannabis, cocaine, hallucinogens, inhalants, opioids, or phencyclidine; personality disorder of the paranoid type; and personality disorder of the schizoid type.

Examples of anxiety disorders that can be treated according to the present invention include, but are not limited to, panic disorder; agoraphobia; a specific phobia; social phobia; obsessive-compulsive disorder; post-traumatic stress disorder; acute stress disorder; and generalized anxiety disorder.

This invention also provides a method of treating a movement disorder selected from Huntington's disease and dyskinesia associated with dopamine agonist therapy in a mammal, including a human, which method comprises administering to said mammal an amount of a selective PDE10 inhibitor effective in treating said disorder.

This invention also provides a method of treating a movement disorder selected from Huntington's disease and dyskinesia associated with dopamine agonist therapy in a mammal, including a human, which method comprises administering to said mammal an amount of a selective PDE10 inhibitor effective in inhibiting PDE10.

This invention further provides a method of treating a movement disorder selected from Parkinson's disease and restless leg syndrome in a mammal, including a human, comprising administering to said mammal an amount of a selective PDE10 inhibitor effective in treating said disorder.

This invention also provides a method of treating a movement disorder selected from Parkinson's disease and restless leg syndrome in a mammal, including a human, comprising administering to said mammal an amount of a selective PDE10 inhibitor effective in inhibiting PDE10.

This invention further provides a method of treating a drug addiction, for example an alcohol, amphetamine, cocaine, or opiate addiction, in a mammal, including a human, which method comprises administering to said mammal an amount of a selective PDE10 inhibitor effective in treating drug addiction.

This invention also provides a method of treating a drug addiction, for example an alcohol, amphetamine, cocaine, or opiate addiction, in a mammal, including a human, which method comprises administering to said mammal an amount of a selective PDE10 inhibitor effective in inhibiting PDE10.

A "drug addiction", as used herein, means an abnormal desire for a drug and is generally characterized by motivational disturbances such a compulsion to take the desired drug and episodes of intense drug craving.

This invention further provides a method of treating a disorder comprising as a symptom a deficiency in cognition in a mammal, including a human, which method comprises administering to said mammal an amount of a selective PDE10 inhibitor effective in treating a deficiency cognition.

This invention also provides a method of treating a disorder comprising as a symptom a deficiency in cognition in a mammal, including a human, which method comprises administering to said mammal an amount of a selective PDE10 inhibitor effective in inhibiting PDE10.

The phrase "deficiency in cognition" as used herein in "disorder comprising as a symptom a deficiency in cognition" refers to a subnormal functioning in one or more cognitive aspects such as memory, intellect, or learning and logic ability, in a particular individual relative to other individuals within the same general age population. "Deficiency in cognition" also refers to a reduction in any particular individual's functioning in one or more cognitive aspects, for example as occurs in age-related cognitive decline.

Examples of disorders that comprise as a symptom a deficiency in cognition that can be treated according to the present invention are dementia, for example Alzheimer's disease, multi-infarct dementia, alcoholic dementia or other drug-related dementia, dementia associated with intracranial tumors or cerebral trauma, dementia associated with Huntington's disease or Parkinson's disease, or AIDS-related dementia; delirium; amnestic disorder; post-traumatic stress disorder; mental retardation; a learning disorder, for example reading disorder, mathematics disorder, or a disorder of written expression; attention-deficit/hyperactivity disorder; and age-related cognitive decline.

This invention also provides a method of treating a mood disorder or mood episode in a mammal, including a human, comprising administering to said mammal an amount of a selective PDE10 inhibitor effective in treating said disorder or episode.

This invention also provides a method of treating a mood disorder or mood episode in a mammal, including a human, comprising administering to said mammal an amount of a selective PDE10 inhibitor effective in inhibiting PDE10.

Examples of mood disorders and mood episodes that can be treated according to the present invention include, but are not limited to, major depressive episode of the mild, moderate or severe type, a manic or mixed mood episode, a hypomanic mood episode; a depressive episode with atypical features; a depressive episode with melancholic features; a depressive episode with catatonic features; a mood episode with postpartum onset; post-stroke depression; major depressive disorder; dysthymic disorder; minor depressive disorder; premenstrual dysphoric disorder; post-psychotic depressive disorder of schizophrenia; a major depressive disorder superimposed on a psychotic disorder such as delusional disorder or schizophrenia; a bipolar disorder, for example bipolar I disorder, bipolar II disorder, and cyclothymic disorder.

As used herein, the term "selective PDE10 inhibitor" refers to a substance, for example an organic molecule, that effectively inhibits an enzyme from the PDE10 family to a greater extent than any other PDE enzyme, particularly any enzyme from the PDE 1-9 families or any PDE11 enzyme. In one embodiment, a selective PDE10 inhibitor is a substance, for example organic molecule, having a Kᵢ for inhibition of PDE10 that is less than or about one-tenth the Kᵢ that the substance has for inhibition of any other PDE enzyme. In other words, the substance inhibits PDE10 activity to the same degree at a concentration of about one-tenth or less than the concentration required for any other PDE enzyme.

In general, a substance is considered to effectively inhibition PDE10 activity if it has an IC₅₀ of less than or about 10µM, preferably less than or about 0.1µM.

A "selective PDE10 inhibitor" can be identified, for example, by comparing the ability of a substance to inhibit PDE10 activity to its ability to inhibit PDE enzymes from the other PDE families. For example, a substance may be assayed for its ability to inhibit PDE10 activity, as well as PDE1, PDE2, PDE3A, PDE4A, PDE4B, PDE4C, PDE4D, PDE5, PDE6, PDE7, PDE8, PDE9, and PDE11.

In one embodiment of the therapeutic methods of the invention described above, the selective PDE10 inhibitor is papaverine.

This invention also provides a method of selectively inhibiting PDE10 in a mammal, including a human, comprising administering to said mammal papaverine in an amount effective in inhibiting PDE10.

The term "treating", as in "a method of treating a disorder", refers to reversing, alleviating, or inhibiting the progress of the disorder to which such term applies, or one or more symptoms of the disorder. As used herein, the term also encompasses, depending on the condition of the patient, preventing the disorder, including preventing onset of the disorder or of any symptoms associated therewith, as well as reducing the severity of the disorder or any of its symptoms prior to onset. "Treating" as used herein refers also to preventing a recurrence of a disorder.

For example, "treating schizophrenia, or schizophreniform or schizoaffective disorder" as used herein also encompasses treating one or more symptoms (positive, negative, and other associated features) of said disorders, for example treating, delusions and/or hallucination associated therewith. Other examples of symptoms of schizophrenia and schizophreniform and schizoaffecctive disorders include disorganized speech, affective flattening, alogia, anhedonia, inappropriate affect, dysphoric mood (in the form of, for example, depression, anxiety or anger), and some indications of cognitive dysfunction.

The term "mammal", as used herein, refers to any member of the class "Mammalia", including, but not limited to, humans, dogs, and cats.

### Brief Description of the Figures

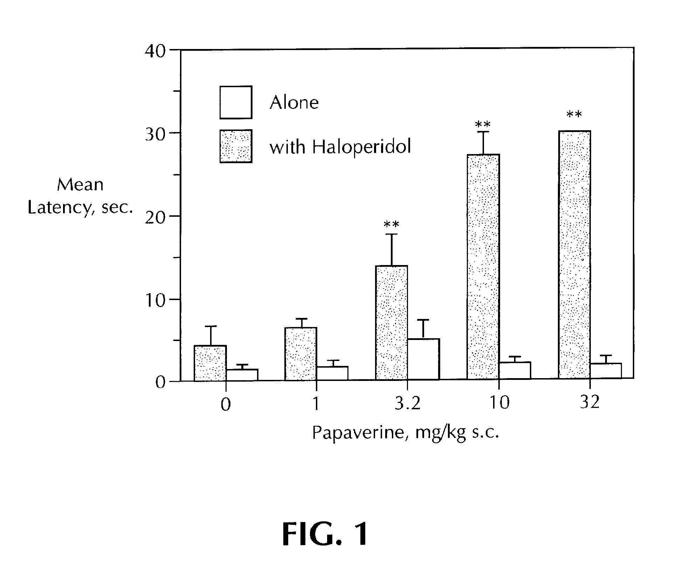

**Figure 1:** The Figure is a bar graph showing catalepsy in animals versus increasing dose of papaverine. The gray bars represent a papaverine in combination with haloperidol and show the potentiation of haloperidol-induced catalepsy by papaverine. The black bars represent papaverine alone. These black bars show that papaverine did not alone induce catalepsy at a dose of up to 32 mg/kg. More particularly, papaverine was administered at the indicated doses either alone or with haloperidol (0.32 mg/kg) 30 min prior to testing. Each bar is the mean latency for six similarly treated animals to remove both forepaws from an elevated bar. Kruskall-Wallace analysis of variance was used to compare the ranked latencies for papaverine alone versus plus haloperidol. Post hoc analysis indicates that animals dosed with 3.2, 10, and 32 mg/kg papaverine plus haloperidol had significantly (**) longer latencies than that of animals treated with haloperidol alone.

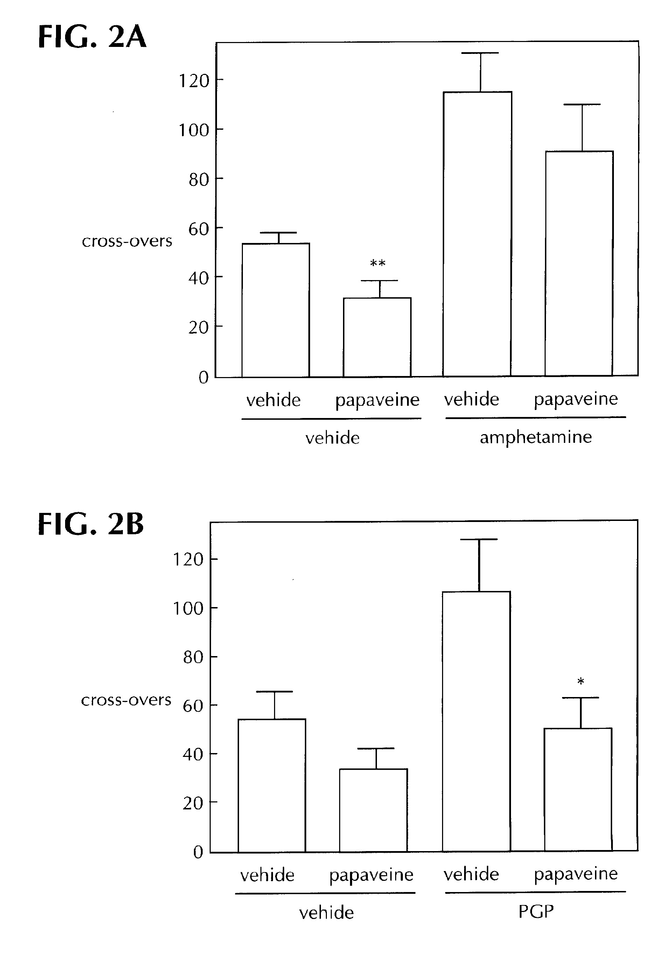

**Figure 2:** The Figure is two bar graphs each showing the mean + SEM number of crossovers for animals in a shuttle box study for the first 60 minutes following substance administration. The top graph compares papaverine's effects on movement alone to papaverine's effects on amphetamine-induced movement. The bottom graph compares papaverine's effects on movement alone to papaverine's effects on PCP-induced movement. Amphetamine was administered at 1 mg/kg, i.p. PCP was administered at 3.2 mg/kg, i.p. Papaverine was co-administered with either agent at a dose of 32 mg/kg, i.p. Data represents the mean + SEM crossovers for the first 60 min following drug administration for n-=8 rats/group.

** p<0.01 versus vehicle/vehicle control; * p<0.05 versus vehicle/PCP by Students t-test

### Detailed Description of the Invention

PDEs 2, 3 and 5, isozymes, including human PDEs, can, for example, be prepared from corpus cavernosum; PDE1, isozymes including human, from cardiac ventricle; and PDE4, isozymes, including human, from skeletal muscle. PDE6 can be prepared, for example, from canine retina. Description of enzyme preparation from native tissue is described, for example, by Boolell, M. et al., Int. J. Impotence Research 8:7-52, 1996, incorporated herein by reference.

PDEs 7-11 can similarly be prepared from native tissue. Isozymes from the PDEs 7-9 and 11 families can alternatively be generated from full length human recombinant clones transfected into, for example, SF9 cells as described in Fisher, D.A., et al., Biochem. Biophys. Res. Comm. 246, 570-577, 1998; Soderling, S.H. et al., PNAS 96: 7071-7076, 1999; Fisher, D.A. et al., J. Biol. Chem. 273, 15559-15564, 1998b; and Fawcett, L., et al., PNAS 97: 3702-3707, 2000; respectively. PDE10 can also be generated from a rat recombinant done transfected into SF9 cells (Fujishige et al., European Journal of Biochemistry, Vol. 266, 1118-1127 (1999)). The enzymes are then prepared by FPLC from the soluble fraction of cell lysates as described for PDE6. The aforementioned references are incorporated in their entireties herein by reference.

In one assay, a substance is screened for inhibition of cyclic nucleotide hydrolysis by the PDE10 and the PDEs from the other gene families. The cyclic nucleotide substrate concentration used in the assay of each individual PDE is 1/3 of the Kₘ concentration, allowing for comparisons of IC₅₀ values across the different enzymes. PDE activity is measured using a Scintillation Proximity Assay (SPA)-based method as previously described (Fawcett et al., 2000). The effect of PDE inhibitors is determined by assaying a fixed amount of enzyme (PDEs 1-11) in the presence of varying substance concentrations and low substrate, such that the IC₅₀ approximates the Kᵢ (cGMP or cAMP in a 3:1 ratio unlabelled to [³H]-Iabeled at a concentration of 1/3 Km). The final assay volume is made up to 100µl with assay buffer [20 mM Tris-HCI pH 7.4, 5 mM MgCl₂, 1 mg/ml bovine serum albumin]. Reactions are initiated with enzyme, incubated for 30-60 min at 30°C to give <30% substrate turnover and terminated with 50 µl yttrium silicate SPA beads (Amersham) (containing 3 mM of the respective unlabelled cyclic nucleotide for PDEs 9 and 11). Plates are re-sealed and shaken for 20 min, after which the beads were allowed to settle for 30 min in the dark and then counted on a TopCount plate reader (Packard, Meriden, CT). Radioactivity units can be converted to percent activity of an uninhibited control (100%), plotted against inhibitor concentration and inhibitor IC₅₀ values can be obtained using the 'Fit Curve' Microsoft Excel extension.

One example of a selective PDE10 inhibitor is papaverine (1-[(3,4-dimethoxyphenyl)methyl]-6,7-dimethoxyisoquinoline ). Papaverine is a known effective smooth muscle relaxant used in the treatment of cerebral and coronary vasospasm as well as for erectile dysfunction. Although the basis of these therapeutic activities is not well understood, they are generally ascribed to papaverine's activity as a nonselective phosphodiesterase inhibitor (The Pharmacological Basis of Therapeutics; Sixth Edition; A.G. Gilman, L.S. Goodman, A. Gilman (eds.) Macmillan Publishing Co., New York, 1980, p. 830). Although papaverine is a naturally occurring plant alkaloid, its complete biosynthesis has been described, for example in Brochmann-Hanssen et al., J. Pharm. Sci. 60:1672, 1971, which is incorporated herein by reference.

A selective PDE10 inhibitor may be administered according to the present invention either alone or in combination with pharmaceutically acceptable carriers, in either single or multiple doses. Suitable pharmaceutical carriers include inert solid diluents or fillers, sterile aqueous solutions and various organic solvents. The pharmaceutical compositions formed thereby can then be readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, methylcellulose, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions containing a selective PDE10 inhibitor in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

A selective PDE10 inhibitor can be administered in the therapeutic methods of the invention orally, transdermally (e.g., through the use of a patch), parenterally (e.g. intravenously), rectally, or topically. In general, the daily dosage of PDE10 inhibitor for treating a disorder or condition according to the methods described herein will generally range from about 0.01 to about 100 mg/kg body weight of the patient to be treated. As an example, a selective PDE10 inhibitor can be administered for treatment of, for example, a psychotic disorder, to an adult human of average weight (about 70kg) in a dose ranging from about 1 mg up to about 7000 mg per day, preferably from about 1 mg to about 1000 mg per day, in single or divided (i.e., multiple) portions. Variations based on the aforementioned dosage ranges may be made by a physician of ordinary skill taking into account known considerations such as the weight, age, and condition of the person being treated, the severity of the affliction, and the particular route of administration chosen.

The following Examples illustrate the present invention. It is to be understood, however, that the invention, as fully described herein and as recited in the claims, is not intended to be limited by the details of the following Examples.

### EXAMPLES

### Example 1. Selective PDE10 Inhibitors: Papaverine:

Papaverine was screened for inhibition of cyclic nucleotide hydrolysis by PDE10 and a battery of PDEs from the other gene families. The cyclic nucleotides substrate concentration used in the assay of each individual PDE was 1/3 of the Km concentration. This allows for comparisons of IC₅₀ values across the different enzymes.

PDE activity was measured using the assay with yttrium silicate SPA beads described above in the Detailed Description section. Radioactivity units were converted to percent activity of an uninhibited control (100%), plotted against inhibitor concentration and inhibitor IC₅₀ values obtained using the 'Fit Curve' Microsoft Excel extension.

We observed that papaverine was an exceptionally potent, competitive inhibitor of PDE10 with an IC₅₀ value of 18 nM (Table 1). Papaverine was considerably less potent against all other PDEs tested. After PDE10, the enzyme inhibited most potently by papaverine was PDE4D with an IC₅₀ of 320 nM, a value 19-fold lower than that for PDE10. Thus, these data reveal for the first time that papaverine is a selective PDE10 inhibitor and that this compound can be used in studies of this enzyme's physiology.

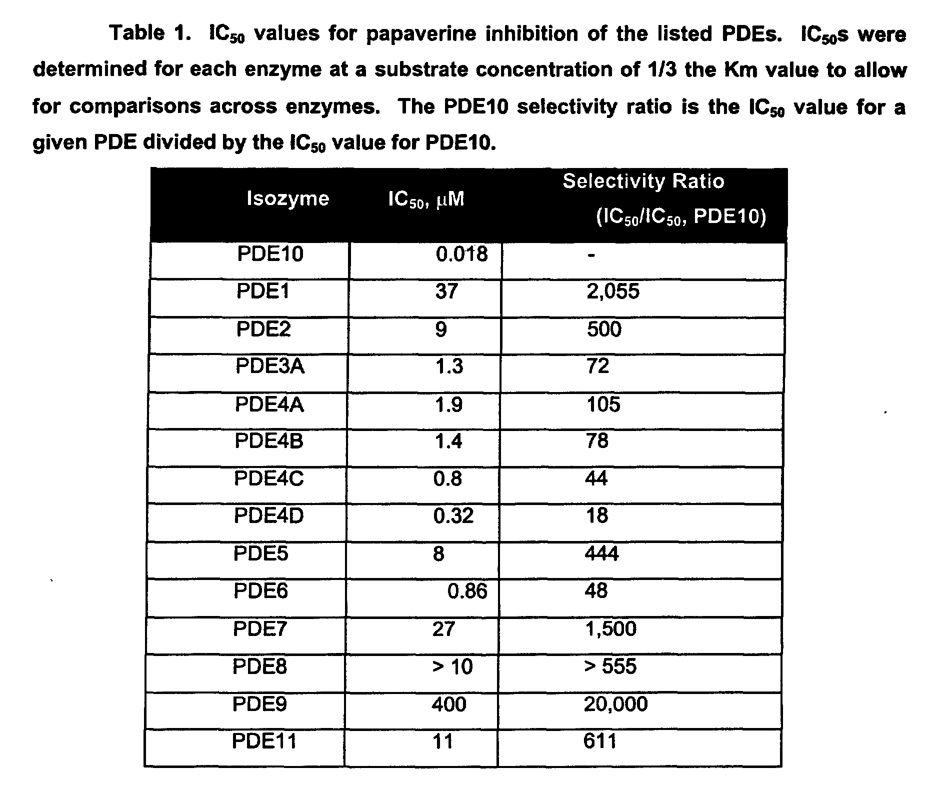

### Example 2. Effects of a Selective PDE10 Inhibitor on Cyclic Nucleotide Metabolism in Medium Spiny Neurons:

We examined the effects of papaverine, a selective PDE10 inhibitor as determined in Example 1, on cyclic nucleotide metabolism in rat medium spiny neurons in primary culture.

Neurons cultured from E17 rat embryo striatum in the presence of BDNF displayed a phenotype very similar to that described previously (Ventimiglia et al., Eur. J. Neurosci. 7 (1995) 213-222 ). Approximately 50 % of these neurons stain positive for GABA immunoreactivity confirming the presence of medium spiny neurons in the cultures. Expression of PDE-10 message in these cultures at 5 DIV was confirmed by RNAase protection assay.

The striatal cultures were prepared as previously described (Ventimiglia et al., Eur. J. Neurosci. 7: 213-222, 1995). Briefly, striata (caudate nucleus and putamen) are dissected from E17 rats, were dissociated to produce a single cell suspension and plated at a density of 5x10⁴ neurons/well in multiwell plates coated with poly-L-ornithine/laminin. The cells were plated in Neurobasal medium with B27 supplements and BDNF (100ng/mL). Experiments were typically performed after 4 days *in vitro.* Medium spiny neurons comprise the majority of cells in these cultures (50 to 60%, as confirmed by GABA immunoreactivity).

For the RNAse protection assay, RNA was prepared from these primary cultures of rat medium spiny neurons by centrifugation at 150, 000 x g at 20°C for 21 hr through a 5.7 M cesium chloride gradient as previously described (Iredale, PA, et al., Mol. Pharmacol.50: 1103-1110, 1996). The RNA pellet was resuspended in 0.3 M sodium acetate, pH 5.2, precipitated in ethanol and the concentration determined by spectrophotometry. The PDE10 riboprobe was prepared by PCR amplification of a 914 bp fragment isolated from mouse cDNA (corresponding to bp 380- bp 1294). This fragment was then cloned into pGEM3Zf. The vector was linearized and T7 RNA polymerase was used to synthesize [³²P]-labeled antisense riboprobe. The RNase protection assay was performed using the RPAII kit (Ambion). Briefly, 5 µg of total cellular RNA was hybridized with [³²P]-labeled PDE10 riboprobe (∼105 cpm/sample) overnight at 42°C. The following day the samples were incubated with RNase A and T1 for 30 min at 37°C and the protected double-stranded RNA fragments were then precipitated and run on a 6% polyacrylamide gel containing urea.

For analyzing effects of papaverine on cyclic nucleotides, the striatal cell cultures, after four days *in vitro,* were washed with Ca²⁺/Mg⁺ free phosphate buffered saline and preincubated for an hour in a buffer containing Ca²⁺/Mg⁺ free phosphate buffered saline, 30mM HEPES, CaCl₂1mM, dextrose 1mg/mL, and MgCl₂ 5mM. The striatal cells were exposed to phosphodiesterase inhibitors and incubated for twenty minutes at 37 degrees Celsius. When measuring cGMP, the neurons were stimulated with sodium nitroprusside, a nitric oxide source for two minutes following the 20-minute incubation with compound. When measuring cAMP, the neurons were stimulated with forskolin, an activator of adenylate cyclase for the duration of the twenty minute compound incubation. The cells were lysed using a 9:1 combination of cAMP SPA direct screening Assay Buffer (0.05M acetate with 0.01% sodium azide) and Buffer A (133mg/mL dodecyltrimethylammonium bromide) and the lysates were frozen on dry ice. A cGMP [1125] or cAMP [1125] scintillation proximity assay (SPA) system (Amersham code RPA 540 and RPA 559, respectively) was used to detect the concentration of the respective cyclic nucleotide in the cell lysate.

Papaverine alone did not effect the low basal level of either CAMP or cGMP in the striatal cultures. We therefore examined the effects of the compound under conditions in which cAMP or cGMP synthesis was stimulated with forskolin or the NO donor sodium nitroprusside (SNP), respectively. Stimulation of the cultures with forskolin (0.1-10 µM) for 20 min resulted in a concentration-dependent increase in cAMP levels. Similarly, brief exposure of the cultures to SNP (3-1000 µM) for 2 min resulted in a concentration-dependent increase in cGMP levels. Forskolin alone (10 µM) did not alter cGMP concentrations nor did SNP (300 µM) increase cAMP levels. In order to determine the effects of papaverine on cAMP and cGMP metabolism, striatal cultures were incubated with various concentrations of the compound and then stimulated with submaximally effective concentrations of either forskolin (1 µM) or SNP (100 µM). These concentrations of forskolin or SNP caused a 2-3 fold increase over basal in cAMP and cGMP, respectively. Papaverine caused a concentration-dependent increase in SNP-induced cGMP accumulation with an EC₂₀₀ (concentration of the inhibitor yielding a 2-fold increase) value of 11.7 µM (Table 2). A maximal effect was observed at 100 µM, at which cGMP levels were elevated 5-fold over that in cultures stimulated with SNP alone. Papaverine also caused an increase in cAMP accumulation in forskolin-stimulated cultures. However, the compound was 3.3-fold less potent at promoting an increase in cAMP than for cGMP. The effects of papaverine in the striatal cultures were compared to other PDE inhibitors with different selectivities (Table 2). IBMX, a nonselective inhibitor caused a concentration dependent (3-100 µM) increase in both cGMP and cAMP accumulation in SNP- or forskolin-stimulated cultures with EC₂₀₀ values of 19 and 30 µM, respectively. The selective PDE4 inhibitor rolipram increased forskolin stimulated CAMP accumulation with an EC₂₀₀ value of 2.5 µM and required 10-fold higher concentrations to double the rate of cGMP accumulation. Zaprinast, an inhibitor of cGMP preferring PDEs, doubled the cAMP levels in these neurons at a concentration of 98 µM. However, 100 µM of this compound did not quite double the level of cGMP. These data reveal for the first time that papaverine has a unique effect on cyclic nucleotide regulation in medium spiny neurons and that this effect is due to the selectivity for PDE10.

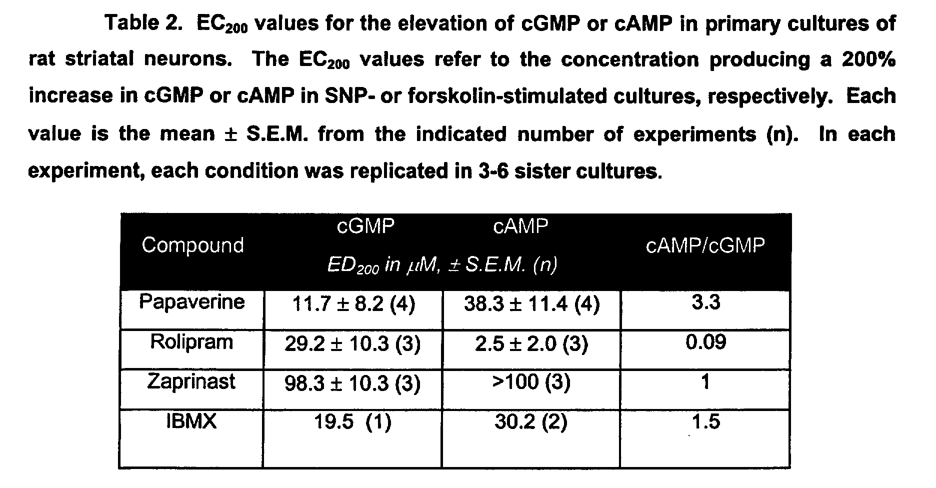

### Example 3. Effect of A Selective PDE 10 Inhibitor in Animal Model of basal ganglia function:

Studies in human and non-human mammals indicate that the basal ganglia regulate a range of motor as well as cognition and emotional/appetitive behaviors (Graybiel, A.M. Current Biology 10 (14):R509-11, 2000). Experimental models in rodents have been developed which can be used to assess the effects of compounds on basal ganglia function. We find that papaverine has an unanticipated unique profile of behavioral effects in two such models.

The effect of papaverine alone and in combination with haloperidol was tested for the ability to induce catalepsy in male CD rats. This animal model is used to analyze the effects of compounds on basal ganglia output. Papaverine (1.0, 3.2, 10, or 32 mg/kg.) or vehicle was administered subcutaneously. For some experiments, this was immediately followed by 1.0 mg/kg haloperidol (s.c. in 0.3% tartaric acid). Thirty minutes after drug administration(s), the degree of catalepsy was quantified by placing the animals forepaws on an elevated (10 cm) bar (1 cm diameter) and determining the latency to remove both forepaws from the bar with a latency cutoff of 30 sec. Latencies were ranked within each treatment group for comparison by a Kruskall-Wallace analysis of variance. Post hoc analysis was by the Mann Whitney U test.

The antipsychotic agent haloperidol produces robust catalepsy in this model, as previously described (Chartoff, E et al., J Pharmacol. Exp. Ther. 291:531-537, 1999). A maximally effective dose of haloperidol was found to be 1 mg/kg, s.c. In contrast, papaverine did not induce catalepsy when administered alone at a dose of up to 32 mg/kg s.c. (p = 0.86). However as shown in Figure 1, papaverine potentiated the cataleptic effect of a submaximal (0.32 mg/kg, s.c.) dose of haloperidol (p<0.001). The minimum effective dose of papaverine for potentiation of haloperidol-induced catalepsy is 3.2 mg/kg, s.c. This experiment demonstrated that papaverine can alter basal ganglia output in a direction consistent with antipsychotic activity.

### Example 4. Effect of A Selective PDE 10 Inhibitor in Animal Model for Psychosis:

We next examined the effect of papaverine on locomotor activity in rats as measured in a shuttle box. Reduction of PCP-stimulated locomotion in rodents is accepted as a primary screen in the search for novel antipsychotic agents. Newer atypical antipsychotic agents generally demonstrate a preferential inhibition of PCP- versus amphetamine-stimulated locomotor activity. Adult, male, Sprague-Dawley rats (250-300 g) were obtained from Charles River (Wilmington, MA). Locomotor activity was assessed using crossover behavior in commercially available shuttle boxes (Coulboum Instruments, Allentown, PA). Data was collected in 5 minute intervals for 1 hour after drug administration. Animals received either vehicle (5% DMSO, 5% Emulphor, 90% Saline) phencyclidine (PCP, Sigma Chem. Co..) or amphetamine Sulfate (RBI) followed immediately by either vehicle or test compound. Statistical analysis was performed using the Student's t-test.

The psychostimulants amphetamine and phencyclidine (PCP) both produce a robust increase in locomotor activity in this model. Papaverine alone (32 mg/kg, i.p.) produced a small decrease in locomotor activity which was statistically significant in some studies (Figure 2). However, this same dose of papaverine produced a significant reduction in the locomotor activity stimulated by 3.2 mg/kg, i.p. phencyclidine without effecting that produced by a behaviorally equivalent dose of amphetamine (1 mg/kg, i.p.).

### Discussion

As noted above, a high expression of PDE10 mRNA in striatum, nucleus accumbens, and olfactory tubercle using *in situ* hybridization has already been reported (Seeger, T. F. Et al., *supra*). We have also found using monoclonal antibody for PDE10 protein, a correspondingly high level of PDE10 protein in these brain regions. Within the striatum and n. accumbens, we found PDE10 mRNA expressed at high levels in the medium spiny neurons. Medium spiny neurons are the output neurons of the striatum, n. accumbens, and olfactory tubercle; and represent approximately 95 % of all the neurons in these brain structures. Furthermore, a high level of PDE10 protein was observed in the projections (axons and terminals) of medium spiny neurons projecting from the striatum, n. accumbens, and olfactory tubercle into other brain regions, including the globus pallidus and substantia nigra. These latter brain regions themselves have low or undetectable levels of PDE10 mRNA. Therefore, the high level of PDE10 protein in these regions arises from the axons and terminals of the medium spiny neurons. In addition, our studies demonstrated PDE10 mRNA and protein expressed at lower levels in neurons of other brain regions, namely the cortex, hippocampus and cerebellum.

The high levels of PDE10 expression in the striatum and nucleus accumbens are particularly interesting given that these are the major cortical input nuclei of the basal ganglia as well as the principal terminal fields for the midbrain dopaminergic projections. The striatum and its ventral extension, the nucleus accumbens, receive glutamatergic afferents from virtually every region of the cerebral cortex and function as a subcortical integration site for a wide range of cortical activities. The dorsal striatum is generally considered to be involved in the regulation of motor behavior whereas the ventral regions, including the accumbens, function in the regulation of emotional/appetitive behaviors. Thus, we believe that PDE10 is likely to be involved in signaling pathways that regulate a number of these basic physiological processes.

Cortical input to the striatum provides the primary excitatory drive for the GABAergic medium spiny projection neurons (MSN) which make up 95% of all striatal neurons. Glutamatergic activation of the MSN is in turn regulated by the massive dopaminergic input from the midbrain. The antagonistic nature of these two afferent systems has been demonstrated in numerous studies. For example, locomotor stimulant activity in laboratory animals can be produced by either dopamine receptor agonists or antagonists of the NMDA subtype of the glutamate receptor (Carlsson, M.L. and Carlsson, A. Trends Neurosci.13:272-276, 1990). The cataleptic effect of D₂ dopamine receptor antagonists such as haloperidol is reduced by NMDA receptor antagonists as is haloperidol-induced gene expression (Chartoff, E et al., J Pharmacol. Exp. Ther. 291:531-537, 1999). More recently, it has been demonstrated that the blockade of D₂ dopamine receptors results in an increase in the phosphorylated or activated state of striatal NMDA receptors (Leveque et al., Journal of Neuroscience 20(11):4011-4020, 2000).

The recognition that all clinically effective antipsychotics possess potent D₂ antagonist activity lead to the original hypothesis that the symptoms of schizophrenia are the result of excessive activity in the mesolimbic dopamine system. The ability of a chemical compound to reduce the stimulant properties of direct or indirect dopamine agonists became an important laboratory test in the search for new antipsychotic agents. More recently, the ability of NMDA receptor antagonist such as PCP to faithfully reproduced the positive, negative and cognitive symptoms of schizophrenia in man (Luby et al., 1959; Rosenbaum et al, 1959; Krystal et al. 1994) has lead to the development of the hypofrontality theory of schizophrenia. Simply put, this hypothesis proposes that striatally-mediated behavioral inhibition is deficient in schizophrenia as a consequence of reduced glutamatergic and specifically, NMDA receptor-mediated, neurotransmission. This hypothesis is entirely consistent with the known antipsychotic effect of D₂ dopamine receptor antagonists given their ability to disinhibit directly or indirectly cortical input to the striatum (as described above). The fidelity with which PCP replicates the symptoms of schizophrenia in humans has lead to the use of PCP-stimulated locomotion in rodents as a primary screen in the search for novel antipsychotic agents. The demonstration that newer and presumably more efficacious atypical antipsychotic agents demonstrate preferential activity against PCP- over amphetamine-stimulated locomotor activity would appear to supports this approach (Gleason S.D. and Shannon H.E. Psychopharmacol. 129:79-84, 1997).

Although current approaches to antipsychotic therapy generally target membrane receptors, we propose here that intracellular manipulations of PDE10 which will enhance NMDA receptor or reduce dopamine D₂ receptor effects within the MSN also can produce antipsychotic effects. Increases in cAMP and PKA activity are known to enhance the response of striatal neurons to glutamate agonists including NMDA (Colwell, C.S. and M.S. Levine, J Neuroscience 15(3)1704-1713, 1995). The neuroleptic action of haloperidol is also dependent on increases in cAMP levels (Ward, R.P. and D.M. Dorsa, Neuroscience 89(3):927-938, 1999) and PKA activation (Adams, M.R. et al., Proc Natl Acad Sci USA 94:12157-12161, 1997). Striatal cGMP levels are also increased after D₂ receptor blockade (Altar, C. A. et al., Eur J. Pharmacol. 181:17-21, 1990), and PKG is known to phosphorylate some of the same downstream substrates as PKA, including the endogenous inhibitor of protein phosphatase I, DARP (Greengard P et al., Brain Res. Rev. 26:274-284, 1998). Therefore, we hypothesized that agents able to selectively increase cyclic nucleotide levels in MSN in the striatum could reasonably be expected to augment striatal function with a resulting antipsychotic effect, and that a PDE10 inhibitor will have therapeutic efficacy in the treatment of psychosis because such a compound will inhibit the PDE10 catalyzed metabolism of cAMP and cGMP, increasing the levels of these cyclic nucleotides in the MSNs.

The experiments with papaverine described above reveal that a selective PDE10 inhibitor can increase cyclic nucleotide concentrations in the MSNs in the striatum of the basal ganglia. The studies in animal models described above show that a selective PDE 10 inhbitor can produce unique and unexpected alterations in basal ganglia function.

In addition to psychosis, abnormal function of the basal ganglia has been implicated in a variety of neuropsychiatric conditions including attention-deficit/hyperactivity disorder (ADHD) and related attentional disorders (Seeman, P. et al., Molecular Psychiatry 3:386-96, 1998), depression (Kapur, S., Biol. Psychiatry 32:1-17, 1992; Willner, P., Brain Res. 287:225-236, 1983) and substance abuse (Self, D.W. Annals of Med. 30:379-389, 1998). Several neurological disorders including Parkinson's disease, restless leg syndrome (Hening, W. et al., Sleep 22:970-999, 1999) and Huntington's disease are also linked to basal ganglia dysfunction. Thus, based on our studies described herein, we believe that a PDE10 inhibitor will have a therapeutic impact on such disorders.

As disclosed above, we have found PDE10 mRNA and protein expressed also in neurons of the hippocampus and cortex. Since cognitive processes are dependant on hippocampus and cortex functioning, we believe that PDE10 also plays a role in cognitive processes and that a PDE10 inhibitor may be used to treat disorders having a characteristic component of deficient cognitive function, such as Alzheimer's disease and age-related cognitive decline (ARCD).

## Claims

1. Use of PDE₁₀ inhibitor in the preparation of a medicament for treating anxiety, or psychotic disorder, or a movement disorder selected from Huntington's disease and dyskinesia associated with dopamine agonist therapy, or a movement disorder selected from Parkinson's disease and restless leg syndrome, or treating a drug addiction, for example an alcohol, amphetamine, cocaine, or opiate addiction, or a disorder comprising as a symptom a deficiency in cognition in a mammal, or a mood disorder or mood episode.

2. Use according to claim 1, wherein the psychotic disorder is selected from schizophrenia, for example of the paranoid, disorganized, catatonic, undifferentiated, or residual type; schizophreniform disorder; schizoaffective disorder, for example of the delusional type or the depressive type; delusional disorder; substance-induced psychotic disorder, for example psychosis induced by alcohol, amphetamine, cannabis, cocaine, hallucinogens, inhalants, opioids, or phencyclidine; personality disorder of the paranoid type; and personality disorder of the schizoid type; and the anxiety disorder is selected from panic disorder; agoraphobia; a specific phobia; social phobia; obsessive-compulsive disorder; post-traumatic stress disorder; acute stress disorder; and generalized anxiety disorder.

3. Use according to claim 1 wherein the disorder comprising as a symptom a deficiency in cognition in a mammal is selected from dementia, for example Alzheimer's disease, multi-infarct dementia, alcoholic dementia or other drug-related dementia, dementia associated with intracranial tumors or cerebral trauma, dementia associated with Huntington's disease or Parkinson's disease, or AIDS-related dementia; delirium; amnestic disorder; post-traumatic stress disorder; mental retardation; a learning disorder, for example reading disorder, mathematics disorder, or a disorder of written expression; attention-deficit/hyperactivity disorder; and age-related cognitive decline.

4. Use according to claim 1 wherein the mood disorder or mood episode is selected from a major depressive episode of the mild, moderate or severe type, a manic or mixed mood episode, a hypomanic mood episode; a depressive episode with atypical features; a depressive episode with melancholic features; a depressive episode with catatonic features; a mood episode with postpartum onset; post-stroke depression; major depressive disorder; dysthymic disorder; minor depressive disorder; premenstrual dysphoric disorder; post-psychotic depressive disorder of schizophrenia; a major depressive disorder superimposed on a psychotic disorder such as delusional disorder or schizophrenia; a bipolar disorder, for example bipolar I disorder, bipolar II disorder, and cyclothymic disorder.

5. Use according to any one of the preceding claims wherein the PDE₁₀ inhibitor has a IC₅₀ against PDE₁₀ of less than 0.1 micromolar.

6. Use according to any one of the preceding claims wherein the PDE₁₀ inhibitor has a selectivity of PDE₁₀ to any of PDE₁ to PDE₁₁ (as defined on page 10 herein) of at least 10:1.

7. Use according to claim 6 wherein the selectivity of PDE₁₀ to any of PDE₁ to PDE₁₁ is at least 15:1.

8. Use according to claims 5 to 7 wherein the PDE₁₀ inhibitor has an IC₅₀ against PDE₁₀ of less than 0.1 micromolar and a selectivity for PDE₁₀ over any of PDE₁ to PDE₁₁ of at least 10:1.

9. Use according to any of one of the preceding claims wherein the PDE₁₀ inhibitor is papaverine.
